# EUROPÄISCHE PATENTANMELDUNG

(11) **EP 0 726 253 A1**
(43) Veröffentlichungstag der Anmeldung: **14.08.1996**
(21) Anmeldenummer: 96101301.8
(22) Anmeldetag: 31.01.1996
(51) Int. Cl.: C07D 207/452, C07D 207/40

(54) **Verfahren zur Herstellung von N-(ortho-Alkylphenyl)-imiden**

(30) Priorität: 13.02.1995 DE 19504626
(71) Anmelder: BAYER AG, 51368 Leverkusen (DE)
(72) Erfinder: Groth, Torsten, Dr., D-51061 Köln (DE); König, Bernd-Michael, Dr., D-51467 Bergisch Gladbach (DE); Käsbauer, Josef, Dr., D-42929 Wermelskirchen (DE); Schwamborn, Michael, Dr., D-51069 Köln (DE)

(57) **Zusammenfassung**

Bei einem besonders vorteilhaften Verfahren zur Herstellung von N-(ortho-Alkylphenyl)-imiden werden cyclische Säureanhydride mit Anilinderivaten umgesetzt, wobei man ohne Zusatz von Polymerisationsinhibitoren und ohne Zusatz von dipolar-aprotischen Lösungsmitteln arbeitet und bei 100 bis 200°C das Reaktionswasser ausschleust.

## Beschreibung

Die vorliegende Erfindung betrifft ein besonders vorteilhaftes Verfahren zur Herstellung von N-(ortho-Alkylphenyl)-imiden, die für die Herstellung von temperaturstabilen Kunststoffen und als Zwischenprodukte für Pharmazeutika und Pflanzenschutzmittel verwendet werden können.

Es ist bekannt, N-(ortho-Alkylphenyl)-maleinimide durch Umsetzung von entsprechend substituierten Anilinen mit Maleinsäureanhydrid (MSA) und Cyclisierung der dabei entstehenden N-substituierten Maleinamidsäuren mit Kondensationsmitteln, z.B. Acetanhydrid, herzustellen (siehe z.B. US-A 3 890 270). Der Einsatz von Kondensationsmitteln ist jedoch unwirtschaftlich und führt zu Nebenprodukten, die auf komplizierte Weise abgetrennt und entsorgt oder zurückgeführt werden müssen. Zudem erfolgt die Aufarbeitung durch Zugabe von Wasser, was zu organisch belasteten Abwässern führt, die in aufwendiger Weise entsorgt werden müssen.

Weiterhin ist bekannt, N-Phenylmaleinimid (NPMI) durch Umsetzung von MSA mit Anilin bei erhöhter Temperatur in Gegenwart eines sauren Katalysators und eines inerten, mit Wasser nicht oder nur wenig mischbaren, aber Azeotrope bildenden Lösungsmittels unter Ausschleusung des gebildeten Wassers herzustellen. Die Ausbeuten sind nur dann einigermaßen hoch, wenn man die Reaktion in Gegenwart von Polymerisationsinhibitoren (siehe z.B. EP-A 165 574) und dipolar-aprotischen Lösungsmitteln, z.B. Dimethylformamid (siehe z.B. EP-A 177 031) oder rückgeführtem NPMI (siehe z.B. EP-A 461 096) durchführt. Derartige zusätzliche Maßnahmen bedeuten bei der Herstellung von NPMI einen Fortschritt, sie sind jedoch auch mit verfahrenstechnischen und wirtschaftlichen Nachteilen verbunden, da die Zusatzstoffe erhöhten Aufwand bei ihrer Entsorgung oder Rückführung mit sich bringen. Zudem erfolgt die Aufarbeitung auch hierbei im allgemeinen durch Zugabe von Wasser, was zu den oben angegebenen zusätzlichen Nachteilen führt.

Schließlich ist aus der DE-A 21 00 800 bekannt, daß sich N-(ortho-Alkylphenyl)-maleinimide durch Umsetzung der entsprechend substituierten Aniline mit MSA in Gegenwart von Basen, z.B. Triethylamin, herstellen lassen. Dieses Verfahren ergibt jedoch in der Hauptsache unlösliche, polymere Produkte (siehe vorliegendes Beispiel 10).

Es besteht daher immer noch das Bedürfnis nach einem Verfahren zur Herstellung von N-(ortho-Alkylphenyl)-imiden, bei dem man die geschilderten Nachteile vermeiden und auf verfahrenstechnisch einfache Weise Produkte mit Ausbeuten von über 90 % der Theorie und in Reinheiten von über 95 % erhalten kann.

Es wurde nun ein Verfahren zur Herstellung von N-(ortho-Alkylphenyl)-imiden der Formel 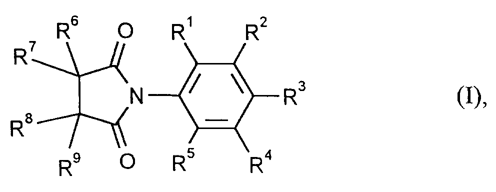 in der
- R¹: für einen C₁-C₆-Alkylrest und
- R² bis R⁹: unabhängig voneinander jeweils für Wasserstoff, gegebenenfalls substituiertes C₁-C₆-Alkyl, gegebenenfalls substituiertes C₂-C₆-Alkenyl, Halogen, NO₂, CN und/oder C₁-C₆-Alkoxy stehen,
wobei R⁶ und R⁷ auch gemeinsam für R¹⁰-CH= mit R¹⁰ = Wasserstoff oder C₁-C₄-Alkyl oder
R⁷ und R⁸ auch gemeinsam für eine kovalente Bindung stehen können,
bei dem man ein cyclisches Säureanhydrid der Formel 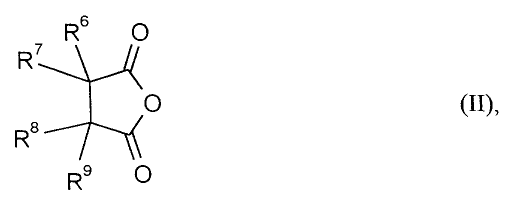 in der
R⁶ bis R⁹ die bei Formel (I) angegebene Bedeutung haben,
mit einem Amin der Formel 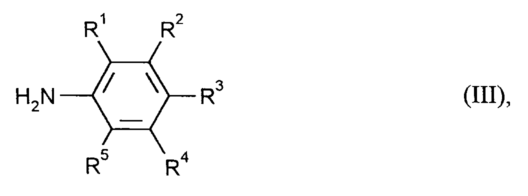 in der
R¹ bis R⁵ die bei Formel (I) angegebene Bedeutung haben,
umsetzt, gefunden, das dadurch gekennzeichnet ist, daß man ohne Zusatz von Polymerisationsinhibitoren und ohne Zusatz von dipolar-aprotischen Lösungsmitteln bei 100 bis 200°C das Reaktionswasser ausschleust.

Soweit R² bis R⁹ für substituierte Alkyl- oder Alkenylreste stehen, kommen als Substituenten z.B. Halogen, NO₂, CN und/oder C₁-C₆-Alkoxy in Frage. Halogen in der Definition von R² bis R⁹ und als Substituent für Alkyl- und Alkenylreste steht beispielsweise für Fluor, Chlor, Brom und/oder Iod, vorzugsweise für Fluor, Chlor und/oder Brom.

In den Formeln (I) und (III) stehen R¹ vorzugsweise für C₁-C₄-Alkyl und R² bis R⁵ unabhängig voneinander vorzugsweise für Wasserstoff, unsubstituiertes geradkettiges oder verzweigtes C₁-C₄-Alkyl, unsubstituiertes geradkettiges oder verzweigtes C₂-C₄-Alkenyl, Chlor, NO₂, CN und/oder C₁-C₆-Alkoxy.

In den Formeln (I) und (II) stehen R⁶ bis R⁹ unabhängig voneinander vorzugsweise für Wasserstoff, unsubstituiertes geradkettiges oder verzweigtes C₁-C₄-Alkyl und/oder unsubstituiertes geradkettiges oder verzweigtes C₂-C₄-Alkenyl oder R⁶ und R⁷ gemeinsam für R¹⁰-CH= mit R¹⁰ = Wasserstoff, Methyl oder Ethyl und R⁸ und R⁹ unabhängig voneinander für Wasserstoff, Methyl, Ethyl und/oder Chlor oder R⁷ und R⁸ gemeinsam für eine kovalente Bindung und R⁶ und R⁹ unabhängig voneinander für Wasserstoff, Methyl, Ethyl und/oder Chlor.

Besonders bevorzugt eingesetzte cyclische Säureanhydride der Formel (II) sind Maleinsäureanhydrid, Itaconsäureanhydrid und Monochlormaleinsäureanhydrid.

Besonders bevorzugt eingesetzte Amine der Formel (III) sind o-Toluidin, 2,3-, 2,4-, 2,5- und 2,6-Dimethylanilin, 2,6-Diethylanilin, 2-Ethyl-6-methylanilin, 2-Isopropylanilin, 2-Chloranilin und 2,6-Diisopropylanilin.

Besonders bevorzugt werden folgende N-(ortho-Alkylphenyl)-imide der Formel (I) hergestellt: N-(2-Methylphenyl)-maleinimid, N-(2-Ethyl-6-methylphenyl)-maleinimid, N-(2,6-Dimethylphenyl)maleinimid, N-(2,6-Diethylphenyl)-maleinimid, N-(2,6-Diisopropylphenyl)-maleinimid, N-(2-Isopropylphenyl)-maleinimid und die entsprechenden Monochlormaleinimide und Itaconimide.

Man kann die erfindungsgemäße Umsetzung gegebenenfalls in Gegenwart von sauren Katalysatoren durchführen. Als saure Katalysatoren kommen z.B. die verschiedensten organischen und anorganischen Protonensäuren sowie saure Ionenaustauscher in Frage. Bevorzugt sind Schwefelsäure, Phosphorsäure, Alkylsulfonsäuren wie Methansulfonsäure, Arylsulfonsäuren wie Benzolsulfonsäure, Toluolsulfonsäuren, Xylolsulfonsäuren und stark und schwach saure Ionenaustauscher in der H-Form. Saure Katalysatoren können z.B. in Mengen von 0 bis 10 Gew.-%, bezogen auf das Säureanhydrid der Formel (II) eingesetzt werden. Vorzugsweise liegt diese Menge bei 0,1 bis 10 Gew.-%, insbesondere bei 0,5 bis 8 Gew.-%.

Man kann die erfindungsgemäße Umsetzung gegebenenfalls in Gegenwart von Lösungsmitteln durchführen, die mit Wasser nicht oder nur wenig mischbar sind, aber mit Wasser Azeotrope bilden können. Derartige Lösungsmittel sind z.B. Toluol, Xylole, Cumol, Mesitylen, Tetralin, Dekalin, Chlorbenzol, Dichlorbenzole, Ethylbenzol, Anisol, Tetrahydrodicyclopentadien und beliebige Mischungen dieser Lösungsmittel. Die Lösungsmittel können dazu dienen, das bei der erfindungsgemäßen Umsetzung entstehende Reaktionswasser durch azeotrope Destillation aus dem Reaktionsgemisch auszuschleusen. Durch Variation des Lösungsmittels und/oder des Drucks kann man die Temperatur verändern, bei der das Wasser azeotrop entfernt wird. Wenn man Lösungsmittel einsetzt, kann dies z.B. in Mengen bis zu 70 Gew.-%, bezogen auf das Reaktionsgemisch (inklusive Lösungsmittel), geschehen. Man kann auch mit relativ wenig oder völlig ohne Lösungsmittel arbeiten, z.B. mit 0 bis 20 Gew.-% Lösungsmittel, bezogen auf das Reaktionsgemisch (inklusive Lösungsmittel, soweit vorhanden).

Das molare Verhältnis von cyclischem Säureanhydrid der Formel (II) zu Amin der Formel (III) kann z.B. 0,5:1 bis 1,5:1 betragen. Vorzugsweise arbeitet man mit äquimolaren Mengen oder einem geringen Überschuß an cyclischem Säureanhydrid, z.B. mit bis zu 1,1 Mol cyclischem Säureanhydrid pro Mol Amin.

Es ist vorteilhaft, das cyclische Säureanhydrid der Formel (II) gegebenenfalls zusammen mit saurem Katalysator und gegebenenfalls zusammen mit Lösungsmittel vorzulegen und das Amin der Formel (III) zuzudosieren. Aus dem cyclischen Säureanhydrid und dem Amin bildet sich zunächst die entsprechende Amidsäure, die sich im allgemeinen schnell unter Wasserabspaltung in das entsprechende N-substituierte Imid der Formel (I) umwandelt.

Die Reaktionstemperatur beim erfindungsgemäßen Verfahren liegt vorzugsweise im Bereich 110 bis 190°C. Der Druck ist nicht kritisch. Man kann bei erhöhtem, erniedrigtem oder normalem Druck arbeiten. Vorzugsweise arbeitet man bei Normaldruck.

Die Ausschleusung des Reaktionswassers kann nicht nur unter Zuhilfenahme eines geeigneten Lösungsmittels durchgeführt werden, wie oben beschrieben, sondern auch durch direktes Abdestillieren aus dem Reaktionsgemisch oder durch Ausblasen mit einem Inertgasstrom.

Das nach der erfindungsgemäßen Umsetzung und der Ausschleusung des Reaktionswassers vorliegende Reaktionsgemisch kann z.B. destillativ aufgearbeitet werden, d.h. man kann das hergestellte N-(ortho-Alkylphenyl)-imid der Formel (I) durch Destillation, vorzugsweise bei vermindertem Druck, gewinnen.

Falls man mit Lösungsmittelzusatz gearbeitet hat, ist es zweckmäßig, das Lösungsmittel vor der Gewinnung des Imids abzutrennen, z.B. durch Abziehen im Vakuum oder Destillation bei normalem oder mäßig erniedrigtem Druck. Das abgetrennte Lösungsmittel kann wiederverwendet werden. Das hinterbleibende Imid kann schon ausreichend rein sein, insbesondere wenn man zuvor eine Entsäuerung (siehe unten) durchgeführt hat.

Es ist vorteilhaft, sauer reagierende Nebenprodukte, überschüssiges cyclisches Anhydrid und/oder saure Katalysatoren vor der Gewinnung des hergestellten Imids durch eine Entsäuerung abzutrennen. Hierzu kann man lösungsmittelhaltiges oder lösungsmittelfreies Reaktionsgemisch mit einer nicht-wäßrigen Base versetzen und den sich bildenden Niederschlag abfiltrieren. Geeignet sind z.B. Ammonium-, Alkali- und/oder Erdalkalicarbonate, Harnstoff, Ammoniumcarbonate und/oder wasserfreies Ammoniak. Die Wasserfreiheit muß nicht absolut sein. Die im Handel als wasserfreie Qualität angebotenen Produkte sind im allgemeinen ausreichend wasserfrei. Es ist vorteilhaft, während und/oder nach Zugabe von nicht-wäßriger Base Temperaturen von beispielsweise 10 bis 100°C, vorzugsweise 20 bis 80°C einzuhalten. Der abgetrennte Niederschlag kann gegebenenfalls gewaschen und die Waschflüssigkeit dann zusammen mit dem Filtrat aufgearbeitet werden. Die weiter obengenannten Lösungsmittel sind für das Waschen des Niederschlages beispielsweise gut geeignet.

Nicht-wäßrige Basen kann man z.B. in Mengen von 0 bis 150 Mol-%, bezogen auf abzutrennende saure Komponenten, einsetzen. Vorzugsweise beträgt diese Menge 50 bis 130 Mol-%.

Das gewonnene N-(ortho-Alkylphenyl)-imid der Formel (I) kann abschließend in beliebiger Weise konfektioniert werden. Beispielsweise kann man es in die Form von Schuppen, Pastillen, Granalien oder eine Lösung, etwa in Styrol oder Acrylnitril, überführen.

Das erfindungsgemäße Verfahren hat eine Reihe von Vorteilen. So benötigt es keine dipolar-aprotischen Cosolventien, auf einen Polymerisationsinhibitor kann bei der Umsetzung verzichtet werden, die Arbeit erfolgt in weitgehend wasserfreiem Milieu und, insbesondere wenn man mit geringen Mengen Lösungsmittel oder lösungsmittelfrei arbeitet, erreicht man hohe Raum-Zeit-Ausbeuten.

Es ist besonders überraschend, daß sich auf die erfindungsgemäße Weise N-(ortho-Alkylphenyl)-imide der Formel (I) auf so einfache Weise mit hohen Ausbeuten und Reinheiten herstellen lassen. Wegen der sterischen Hinderung durch den Rest R¹ war bei den einzusetzenden Aminen der Formel (III) mit einer geringeren Reaktivität zu rechnen und damit mit einer erhöhten Neigung zu Nebenreaktionen. Die bisherige technische Entwicklung auf dem Gebiet der Herstellung von N-Phenyl-imiden führte in jüngerer Zeit zu relativ komplizierten Verfahren. Deshalb hat das relativ einfache erfindungsgemäße Verfahren nicht nahegelegen.

### Beispiele

### Beispiel 1

500 g eines Xylol-Isomerengemisches, 206,0 g Maleinsäureanhydrid und 5,0 g Schwefelsäure (80 %ig) wurden unter Rückfluß (142°C) am Wasserabscheider gerührt. Nach 10 Minuten waren 2 ml Wasser ausgekreist und eine klare Lösung entstanden. Bei 142 bis 150°C wurden in 4 Stunden unter Stickstoffüberlagerung 270,4 g 2-Ethyl-6-methylanilin unter die Oberfläche zudosiert und 38 ml Wasser azeotrop ausgeschleust. Die Lösung wurde 1 Stunde nachgerührt, dabei weitere 2 ml Wasser ausgeschleust, dann auf 60°C abgekühlt, mit 11,0 g festem Ammoniumcarbonat versetzt, 1 Stunde gerührt, über eine Drucknutsche filtriert und das hellgelbe Filtrat am Rotationsverdampfer bei 22 mbar (Badtemperatur: 99°C) eingedampt. Der Rückstand (425 g) wurde nach Zusatz von 122 mg eines Polymerisationsinhibitors (Vulkanox® ZKF) und 350 mg Phosphorsäure (85 %ig) im Vakuum über eine 10 cm-Vigreux-Kolonne destilliert (Kopftemperatur: 125 bis 131°C, Sumpftemperatur: 146 bis 148°C, Druck: 1 mbar). Es wurden 409 g N-(2-Ethyl-6-methylphenyl)-maleinimid (95 % der Theorie) mit einem Gehalt von 99,8 Gew.-% (bestimmt durch HPLC) mit einem Schmelzpunkt von 85°C und einer Säurezahl von weniger als 0,1 mg Kaliumhydroxid/g erhalten.

### Beispiele 2 bis 5

Es wurde verfahren wie in Beispiel 1, jedoch wurden andere Amine eingesetzt und andere N-(ortho-Alkylphenyl)-maleinimide erhalten. Einzelheiten sind aus der nachfolgenden Tabelle ersichtlich.

| Bsp. Nr. | eingesetztes Amin | erhaltenes N-(ortho-Alkylphenyl)-maleinimid | Ausbeute (% d.Th.) | Schmelzpunkt (°C) |
|---|---|---|---|---|
| 2 | o-Toluidin | N-(2-Methylphenyl)-maleinimid | 93 | 72-72,5 |
| 3 | 2,6-Diethylanilin | N-(2,6-Diethylphenyl)-maleinimid | 92 | 73,5-73,8 |
| 4 | 2,6-Dimethylanilin | N-(2,6-Dimethylphenyl)-maleinimid | 92 | 99-100 |
| 5 | 2-Isopropylanilin | N-(2-Isopropylphenyl)-maleinimid | 91 | 99,2-99,4 |

### Beispiel 6

Es wurde verfahren wie in Beispiel 1, jedoch wurde die abschließende Vakuumdestillation nicht durchgeführt. Bei den 425 g Rückstand handelte es sich um N-(2-Ethyl-6-methylphenyl)-maleinimid in einer Ausbeute von 96,6 % der Theorie, mit einem Gehalt von 97,8 Gew.% (bestimmt durch HPLC), einem Schmelzpunkt von 84,5 bis 85°C und einer Säurezahl von weniger als 0,1 mg Kaliumhydroxid/g.

### Beispiel 7

Es wurde verfahren wie in Beispiel 1, jedoch wurde keine Entsäuerung mit Ammoniumcarbonat durchgeführt. Es wurden 410 g N-(2-Ethyl-6-methylphenyl)-maleinimid (95 % der Theorie) mit einem Gehalt von 99,5 Gew.-% (bestimmt durch HPLC), einem Schmelzpunkt von 84,5 bis 85°C und einer Säurezahl von 1,3 mg Kaliumhydroxid/g erhalten.

### Beispiel 8

50 g Toluol, 206,0 g Maleinsäureanhydrid und 2,0 g p-Toluolsulfonsäure-Hydrat wurden vorgelegt und an einem Wasserabscheider auf Rückfluß (135°C) erhitzt. In 4 Stunden wurden unter Stickstoffüberlagerung 270,4 g 2-Ethyl-6-methylanilin unter die Oberfläche eindosiert und dabei 36 ml Wasser abgeschieden. Durch gelegentliche Zugabe von insgesamt 50 g Toluol wurde die im Laufe der Dosierung steigende Siedetemperatur auf maximal 152°C begrenzt. Danach wurde die Lösung noch 1 Stunde nachgerührt, wobei weitere 2 ml Wasser ausgeschleust wurden, dann auf 65°C abgekühlt, mit 11,0 g festem Ammoniumcarbonat versetzt, eine weitere Stunde gerührt, über eine Drucknutsche filtriert und das hellgelbe Filtrat am Rotationsverdampfer bei 22 mbar (Badtemperatur: 99°C) eingedampft. Der Rückstand (416 g) wurde nach Zusatz von 122 mg eines Polymerisationsinhibitors (Vulkanox® ZKF) und 350 mg Phosphorsäure (85 %ig) im Vakuum über eine 10 cm-Vigreux-Kolonne destilliert. Die Destillationsbedingungen waren wie in Beispiel 1 angegeben. Es wurden 396 g N-(2-Ethyl-6-methylphenyl)-maleinimid (91,7 % der Theorie) mit einem Gehalt von 99,7 Gew.-% (bestimmt durch HPLC), einem Schmelzpunkt von 85°C und einer Säurezahl von unter 0,1 mg Kaliumhydroxid/g erhalten.

### Beispiel 9 (Vergleichsbeispiel nach US-A 3 890 270)

196 g Maleinsäureanhydrid wurden bei Raumtemperatur in 2,5 l Diisopropylether gelöst. Innerhalb von 4 Stunden wurde bei Raumtemperatur eine Lösung von 270,4 g 2-Ethyl-6-methyl-anilin im 200 ml Diisopropylether zugetropft, die resultierende Suspension auf 15 bis 20°C abgekühlt, der Feststoff abgesaugt und in eine Mischung aus 670 ml Acetanhydrid und 65 g wasserfreiem Natriumacetat eingetragen. Die entstandene Suspension wurde unter Rühren auf 100°C erhitzt, 30 Minuten bei dieser Temperatur gerührt, dann auf 30°C abgekühlt und in 1,3 l Eiswasser eingetragen. Der Niederschlag wurde abgesaugt, mit Eiswasser und Petrolether gewaschen und getrocknet. Es wurden 321 g N-(2-Ethyl-6-methylphenyl)-maleinimid (92,9 % der Theorie) mit einem Gehalt von 97,8 Gew.-% (bestimmt durch HPLC) und einem Schmelzpunkt von 84,5 bis 85°C erhalten.

### Beispiel 10 (Vergleichsbeispiel nach DE-A 21 00 800)

500 g eines Xylol-Isomerengemischs, 196 g Maleinsäureanhydrid und 0,60 g Triethylamin wurden unter Rückfluß an einem Wasserabscheider gerührt und in 4 Stunden unter Stickstoffüberlagerung mit 240,4 g 2-Ethyl-6-methylanilin versetzt. Die Lösung verfärbte sich zunächst braun, dann tiefrot und es schieden sich große Mengen eines Feststoffs ab, der das Rühren stark erschwerte. Es wurden 24 ml Wasser ausgekreist. Nach dem Abkühlen wurde das erstarrte Reaktionsgemisch (ca. 900 g) analysiert. Es enthielt lediglich 4,4 Gew.-% N-(2-Ethyl-6-methylphenyl)-maleinimid. Die Ausbeute betrug damit nur 9,2 % der Theorie.

## Patentansprüche

1. Verfahren zur Herstellung von N-(ortho-Alkylphenyl)-imiden der Formel 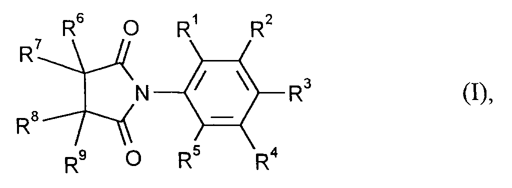 in der
R¹ für einen C₁-C₆-Alkylrest und
R² bis R⁹ unabhängig voneinander jeweils für Wasserstoff, gegebenenfalls substituiertes C₁-C₆-Alkyl, gegebenenfalls substituiertes C₂-C₆-Alkenyl, Halogen, NO₂, CN und/oder C₁-C₆-Alkoxy stehen,
wobei R⁶ und R⁷ auch gemeinsam für R¹⁰-CH= mit R¹⁰ = Wasserstoff oder C₁-C₄-Alkyl oder
R⁷ und R⁸ auch gemeinsam für eine kovalente Bindung stehen können,
bei dem man ein cyclisches Säureanhydrid der Formel 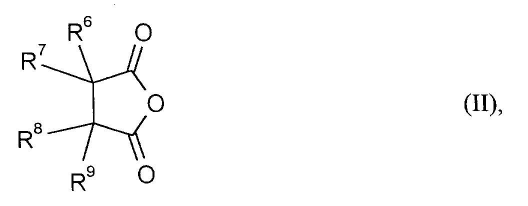 in der
R⁶ bis R⁹ die bei Formel (I) angegebene Bedeutung haben,
mit einem Amin der Formel 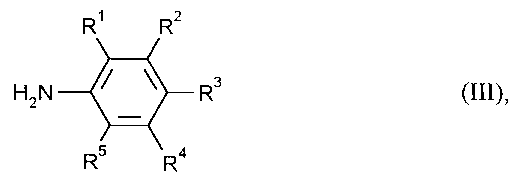 in der
R¹ bis R⁵ die bei Formel (I) angegebene Bedeutung haben,
umsetzt, dadurch gekennzeichnet, daß man ohne Zusatz von Polymerisationsinhibitoren und ohne Zusatz von dipolar-aprotischen Lösungsmitteln bei 100 bis 200°C das Reaktionswasser ausschleust.

2. Verfahren nach Anspruch 1, dadurch gekennzeichnet, daß in den Formeln (I) und (III) R¹ für C₁-C₄-Alkyl und R² bis R⁵ unabhängig voneinander für Wasserstoff, unsubstituiertes geradkettiges oder verzweigtes C₁-C₄-Alkyl, unsubstituiertes geradkettiges oder verzweigtes C₂-C₄-Alkenyl, Chlor, NO₂, CN und/oder C₁-C₆-Alkoxy und in den Formeln (I) und (II) R⁶ bis R⁹ unabhängig voneinander für Wasserstoff, unsubstituiertes geradkettiges oder verzweigtes C₁-C₄-Alkyl und/oder unsubstituiertes geradkettiges oder verzweigtes C₂-C₄-Alkenyl oder R⁶ und R⁷ gemeinsam für R¹⁰-CH= mit R¹⁰ = Wasserstoff, Methyl oder Ethyl und R⁸ und R⁹ unabhängig voneinander für Wasserstoff, Methyl, Ethyl und/oder Chlor oder R⁷ und R⁸ gemeinsam für eine kovalente Bindung und R⁶ und R⁹ unabhängig voneinander für Wasserstoff, Methyl, Ethyl und/oder Chlor stehen.

3. Verfahren nach Ansprüchen 1 und 2, dadurch gekennzeichnet, daß man als cyclisches Säureanhydrid der Formel (II) Maleinsäureanhydrid, Itaconsäureanhydrid oder Monochlormaleinsäureanhydrid und als Amin der Formel (III) o-Toluidin, 2,3-, 2,4-, 2,5- oder 2,6-Dimethylanilin, 2,6-Diethylanilin, 2-Ethyl-6-methylanilin, 2-Isopropylanilin, 2-Chloranilin oder 2,6-Diisopropylanilin einsetzt.

4. Verfahren nach Ansprüchen 1 bis 3, dadurch gekennzeichnet, daß man es in Gegenwart von 0 bis 10 Gew.-% sauren Katalysatoren, bezogen auf das Säureanhydrid der Formel (II), durchführt.

5. Verfahren nach Ansprüchen 1 bis 4, dadurch gekennzeichnet, daß man es in Gegenwart von Lösungsmitteln durchführt, die mit Wasser nicht oder nur wenig mischbar sind, aber mit Wasser Azeotrope bilden können, wobei man solche Lösungsmittel in Mengen bis zu 70 Gew.-%, bezogen auf das Reaktionsgemisch (inklusive Lösungsmittel) einsetzt.

6. Verfahren nach Ansprüchen 1 bis 5, dadurch gekennzeichnet, daß das molare Verhältnis von cyclischem Säureanhydrid der Formel (II) zu Amin der Formel (III) 0,5:1 bis 1,5:1 beträgt.

7. Verfahren nach Ansprüchen 1 bis 6, dadurch gekennzeichnet, daß man das cyclische Säureanhydrid der Formel (II) gegebenenfalls zusammen mit saurem Katalysator und gegebenenfalls zusammen mit Lösungsmittel vorlegt und das Amin der Formel (III) zudosiert.

8. Verfahren nach Ansprüchen 1 bis 7, dadurch gekennzeichnet, daß man das Reaktionswasser unter Zuhilfenahme eines geeigneten Lösungsmittels oder durch direktes Abdestillieren aus dem Reaktionsgemisch oder durch Ausblasen mit einem Inertgasstrom ausschleust.

9. Verfahren nach Ansprüchen 1 bis 8, dadurch gekennzeichnet, daß man das nach der Umsetzung und der Ausschließung des Reaktionswassers vorliegende Reaktionsgemisch destillativ aufarbeitet.

10. Verfahren nach Ansprüchen 1 bis 9, dadurch gekennzeichnet, daß man sauer reagierende Nebenprodukte, überschüssiges, cyclisches Anhydrid und/oder saure Katalysatoren vor der Gewinnung des hergestellten Imids durch eine Entsäuerung mit einer nicht-wäßrigen Base abtrennt, wobei man die nicht-wäßrige Base in Mengen von 0 bis 150 Mol-%, bezogen auf abzutrennende saure Komponenten, einsetzt.
